# EUROPEAN PATENT SPECIFICATION

(11) **EP 4 414 816 B1**
(45) Date of publication and mention of the grant of the patent: **14.10.2026**
(21) Application number: 24185514.7
(22) Date of filing: 12.01.2017
(51) Int. Cl.: A61B 34/35, A61B 34/37, G06F 3/01, A61B 34/30

(54) **UNIFORM SCALING OF HAPTIC ACTUATORS**
EINHEITLICHE SKALIERUNG HAPTISCHER AKTUATOREN
MISE À L'ÉCHELLE UNIFORME D'ACTIONNEURS HAPTIQUES

(30) Priority: 12.01.2016 US 201662277827 P
(43) Date of publication of application: 14.08.2024
(62) Document number(s) of the earlier application(s) in accordance with Art. 76 EPC: 21153588.5 / 3 861 959; 17738964.0 / 3 402 434
(73) Proprietor: Intuitive Surgical Operations, Inc., Sunnyvale, CA 94086 (US)
(72) Inventor: VERNER, Lawton N., Sunnyvale, CA 94086 (US); LIAO, Hsien-Hsin, Sunnyvale, CA 94086 (US)
(74) Representative: MacDougall, Alan John Shaw

(56) References cited:
- WO-A1-2006/120666
- US-A- 4 604 016
- US-A1- 2007 142 968
- US-A1- 2013 289 767
- US-A1- 2015 360 365

## Description

### CLAIM OF PRIORITY

This application claims the benefit of priority to U.S. Provisional Patent Application No. 62/277,827, filed on January 12, 2016.

### FIELD

The present invention is directed towards systems for providing haptic feedback to the operator of a surgical system, and more particularly to maintaining an intuitive haptic profile for the user when haptic feedback actuators reach performance limits.

### BACKGROUND

Tele-operated surgical systems are often intended to enhance surgeon precision and/or reduce patient trauma during a medical procedure. In such systems, a surgeon interacts with input devices (sometimes referred to as "masters" or "master controllers") to control surgical instruments that are actuated by drive mechanisms such as motors. Because the surgeon is not directly manipulating the surgical instruments, in can sometimes be beneficial to provide haptic feedback at the input devices that indicates or replicates interaction forces (e.g., felt at the surgical instruments, other elements of the surgical system, and/or virtual or synthetic elements/features generated by the surgical system). Note that the force feedback being presented to the user may be the sum of the feedback from a sensor, from an algorithm, from a user interface cue, collision detection, model interaction, etc.

To provide a good user experience, the surgeon would ideally experience a seamless haptic experience through system state and configuration changes. However, this can be difficult to accomplish, for example the actuators used to provide the haptic feedback are have different performance limits or if different actuators reach their performance limits at different times. In such instances, the haptic feedback presented to the user may not properly align with the perceived user experience (e.g., actual force being sensed at the surgical instrument or the visual representation of the interactive object), resulting in a confusing or non-intuitive user experience.

For example, motor torque limits (i.e. saturation limits) are typically imposed in software for robotic interfaces. These performance limits can be created for several reasons including to protect the motor from overheating, to limit the force applied to the surgeon/patient, and/or to keep the motor in it's ideal torque operating range. Limiting the torque at the motor can yield non-isotropic force saturation at the interface (e.g., the handle of the input device or the tip of the instrument). This means when you are trying to present a force at the interface that involves two or more motors, the force direction can be incorrect if one motor is restricted by a motor torque limit.

This can be especially problematic when rendering force to the user (haptic feedback) at the input device. The user may be feeling a force in a given direction, but as the force applied to the user increases and reaches a torque limit for any of the motors associated with render the force to the user, the direction of the force displayed to the user begins to rotate which can be disconcerting and/or confusing for the user.

It is therefore desirable to provide a system and method for ensuring haptic feedback that is consistent with the force environment at the surgical instrument.

US 4604016 A discloses a hand controller or cursor for the actuation of slaved apparatus. The cursor including a handle extending from a line attachment member comprised of four equally spaced legs.

WO 2006/120666 A1 discloses a remote control catheterization system comprising: a propelling device, which controllably inserts a flexible, elongate probe into the body of a patient; and a control unit, in communication with the propelling device, and comprising user controls which are operated by a user of the system remote from the patient to control insertion of the probe into the body by the propelling device.

US 2007/142968 A1 discloses a robotic surgical system that has a robot arm holding an instrument for performing a surgical procedure, and a control system for controlling movement of the arm and its instrument according to user manipulation of a master manipulator.

US 2013/289767 A1 discloses an apparatus and a method for controlling a robot that may scale a motion of a surgical robot based on a type of object gripped by the surgical robot.

US 2015/360365 A1 discloses a master motion information obtaining unit obtaining at least one or more pieces of master motion information including a position, a posture, a speed, and an angular velocity of a master arm mechanism. It also discloses a physical information obtaining unit obtaining physical information of an operator including an arm weight of the operator.

### SUMMARY

The present invention provides a surgical system in accordance with claim 1. Other aspects of the invention are disclosed herein in accordance with the dependent claims.

To minimize discrepancies between expected force directions and haptic force feedback directions, the outputs of the haptic feedback actuators are scaled whenever one or more of the actuators reaches a predetermined output threshold, thereby maintaining proper haptic feedback directionality when individual actuators would otherwise be commanded to operate outside their accurate performance range. Such scaling may change the overall haptic feedback magnitude, but allows the haptic feedback direction to be appropriately maintained.

In some embodiments, where software limits the output of any actuator, a monitoring process can determine when an output of an actuator would exceed a maximum threshold output (at or below the software-defined output limit), and at that point scale down the output of at least one of the other
actuators so that the desired direction of the total output (e.g., force or torque) is maintained. In some embodiments, the monitoring process can additionally or alternatively determine when an output of an actuator would fall below a minimum output threshold (below which the actuator output may be too low to generate accurately), and at that point scale up its output and that of at least one of the other actuators so that the desired direction of the total output (e.g., force or torque) is maintained. In some embodiments, all actuators are scaled when one reaches its threshold output, while in other embodiments, only concurrently active actuators are scaled. In some embodiments, the output thresholds for the actuators are fixed, and in various other embodiments, the output thresholds for the actuators can vary over time or based on actuator and/or system status.

### DRAWINGS

FIG. 1 shows a non-claimed method of providing consistent haptic feedback to a user of a surgical system as actuator maximum outputs are reached.
FIGS. 2A and 2B shows an exemplary haptic force vector outputs when actuator output maximums are ignored vs. addressed.
FIG. 3A-3B show an exemplary surgical system providing haptic force feedback responsive to actuator output maximum thresholds, in accordance with various embodiments of the invention.

### DETAILED DESCRIPTION

In the following detailed description of the aspects of the invention, numerous specific details are set forth in order to provide a thorough understanding of the disclosed embodiments.

To avoid needless descriptive repetition, one or more components or actions described in accordance with one illustrative embodiment can be used or omitted as applicable from other illustrative embodiments.

To minimize discrepancies between desired (e.g., sensed or modeled by a surgical system) and actual haptic force feedback directions, the outputs of the haptic feedback actuators are scaled whenever a commanded output of one or more of the actuators falls outside a predetermined working range for that actuator. The predetermined working range can be defined by a maximum output threshold equal to or less than the output limit of the actuator, and/or a minimum output threshold equal to or greater than a minimum accurate output level of the actuator. Such scaling may reduce the overall haptic feedback magnitude, but allows the haptic feedback direction to be appropriately maintained, which is often a more critical aspect of haptic feedback.

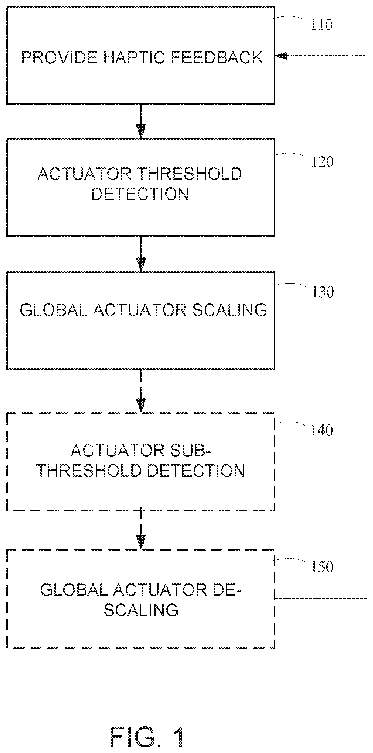

FIG. 1 shows a non-claimed method for providing directionally consistent haptic feedback when actuator output limits are exceeded. In a PROVIDE HAPTIC FEEDBACK step 110, a surgical system that allows a user (e.g., surgeon) to control a surgical instrument (and/or other elements of the surgical system, such as a robotic arm, set up structure, or positioning element such as a boom or cart) via an input device(s) (e.g., lever(s), gripper(s), joystick(s), or any other structure capable of receiving user input), and then provides force feedback to that input device based on a desired haptic feedback profile (a set of one or more haptic feedback effects that at least partially reproduce or represent the physical experience of a real or virtual/modeled interaction). The haptic feedback profile can be based on any haptic model input, such as sensed forces at the instrument (e.g., tissue or other instrument interactions) or robotic arm (e.g., arm collisions with structures or staff), user guidance (e.g., haptic detents, fences, or other profiles to provide guidance for the user to move the input device(s) along a desired path or trajectory), and user interface (UI) elements (e.g., presenting a virtual handle or steering wheel to the user). This haptic feedback can be anything from direct replication of the haptic feedback profile, to scaling of the haptic feedback profile, to applying a nonlinear modification of the haptic feedback profile, or any other transformation (e.g., force scaling that varies depending on one or more other factors such as instrument state/speed, viewing magnification, etc.).

The actual force feedback provided at the input device is generated by two or more actuators (e.g., motors, drives, or any other motive elements) that work cooperatively to provide feedback of varying force and direction. For example, an input device having pitch and yaw capabilities may be coupled to a first pair of actuators that apply forces in opposing directions about the pitch axis, and a second pair of actuators that apply forces in opposing directions about the yaw axis. Two or more of the pitch and yaw actuators can then be used simultaneously to provide force feedback that is offset from the pitch and yaw axes.

Because actuators generally do not have performance characteristics that are exactly the same, whether due to inherent performance limitations or due to operational constraints/effects (e.g., heat, mechanical restrictions). For example, one out of the group of haptic feedback actuators will typically reach its maximum output level before the others. Any commanded output beyond that maximum output level will not result in any increased output, and consequently any haptic feedback that involves the maxed-out actuator will likely deviate from the expected force feedback direction. Additionally, the actuators will may begin producing noisier (less precise) outputs as outputs decrease below a certain level, such that haptic feedback below a certain level may also deviate from the expected force feedback direction.

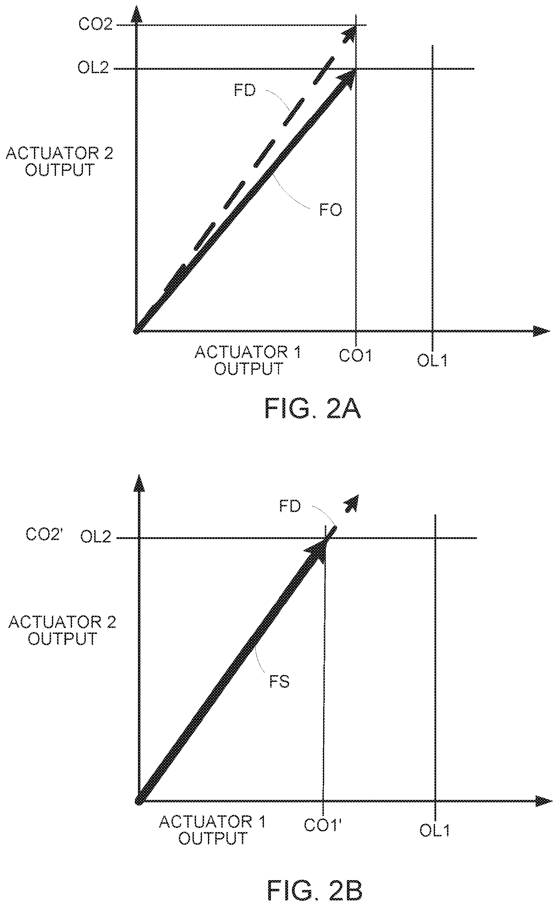

FIG. 2A graphically depicts an example of this haptic offset, with the outputs of first and second actuators ("Actuator 1" and "Actuator 2", respectively) represented by the graph's horizontal and vertical axes, respectively. Also indicated on the axes of the graph are output limits OL1 and OL2 for the first and second actuators, respectively, representing the output limits for the first and second actuators.

As noted above, the actuators cannot exceed their output limits, which may be static (e.g., defined by inherent performance characteristics of the actuators), or dynamic (e.g., based on a current parameter of an actuator, such as temperature, or a physical restriction such as a kinematic configuration of the driven structure that places the actuator in a mechanically unfavorable position).

The problem thus arises if a commanded output of an actuator exceeds its output limit, such as indicated by desired (commanded) feedback force FD. To generate desired feedback force FD, actuator 1 receives a commanded output CO1, and actuator 2 receives a commanded output CO2. Commanded output CO1 is less than output limit OL1, and so can be provided by actuator 1. However, because commanded output CO2 is greater than output limit OL2, the actual output of actuator 2 will be limited to output level OL2, resulting in an overall feedback force FO that is both smaller than, and offset from, desired feedback force FD. While a slight variability in the magnitude of haptic feedback can generally be accommodated by a user without much difficulty, a deviation in force direction can be quite misleading to a user and can result in improper control actions by the user in response.

Returning to FIG. 1, to mitigate this force feedback offset issue, in an ACTUATOR THRESHOLD DETECTION step 120, any commanded output for an actuator that exceeds a maximum output threshold for that actuator is identified. Note that while in some embodiments the maximum output threshold for an actuator can be defined as the output limit for that actuator, in various other embodiments the maximum output threshold could be set at a level below the output limit to provide a buffer for detecting the approach of the actuator limit and/or applying the scaling factor (as described in greater detail below) before any output limits are reached. Note further that as described above, the maximum output thresholds for the haptic feedback actuators can be either static or dynamic, and can be individualized or common across the actuators.

Then, in a GLOBAL ACTUATOR SCALING step 130, a common scaling factor is applied to the commanded outputs for the actuators. The scaling factor is selected to keep the output of the identified actuator (i.e., the actuator identified in step 120 having a commanded output greater than its output threshold) less than its output limit. Because the scaling factor is applied to the commanded output for each actuator, the direction of the overall force is maintained, with a reduced overall magnitude. Note that in some embodiments, if multiple actuators would receive commanded outputs that would exceed their output limits, the scaling factor would be based on the commanded output that exceeds its associated actuator's output threshold by the greatest amount - i.e., the scaling factor would be based on the "worst" output discrepancy.

FIG. 2B graphically depicts an example of this haptic scaling, relative to the same first and second actuator characteristics (output limits OL1 and OL2, respectively) and desired feedback force FD. However, rather than allowing the output limits associated with actuator 2 to pull the overall haptic force off the desired force direction as shown in FIG. 2A, a scaling factor is applied to the commanded outputs CO1 and CO2 to reduce both proportionately to adjusted commanded outputs CO1' and CO2', respectively. The scaling factor is selected such that adjusted commanded output CO2' reduced at least to output limit OL2, although in various other embodiments the scaling factor can be selected to cause adjusted commanded output CO2' to be some increment less than output limit OL2.

In some embodiments, actuators 1 and 2 could additionally or alternatively exhibit reduced output accuracy at low output levels. In such embodiments, a scaling factor could be applied to commanded outputs CO1 and CO2 if either is less than a minimum output threshold for actuator 1 or actuator 2, respectively. The scaling factor would then increase the adjusted commanded outputs CO1' and CO2' above the level at which output accuracy is degraded. Note that as described above, the minimum output thresholds for the haptic feedback actuators can be either static or dynamic, and can be individualized or common across the actuators.

In any event, the application of the scaling factor results in an overall scaled feedback force FS that is aligned directionally with the original desired feedback force FD. As noted above, a consistent haptic experience can be provided even with the change in force magnitude so long as directional consistency of the force feedback is maintained.

Returning to FIG. 1, in various embodiments, when it is detected that the commanded output (unscaled) for the actuator identified in step 120 would go beyond the output threshold (e.g., exceed the maximum output threshold or fall below the minimum output threshold) for that actuator in an optional ACTUATOR SUB-THRESHOLD DETECTION step 140, the scaling factor applied to all commanded outputs in step 130 is removed (or set to 1) in an optional GLOBAL ACTUATOR DE-SCALING step 150. Returning to step 110, unsealed haptic feedback is provided going forward.

As noted previously, in some embodiments, the output thresholds for the haptic feedback actuators can be dynamic - i.e., the specific values can change depending on actuator operational parameters, input device kinematic configuration, or various other factors. In such embodiments, over the course of operation of the surgical system, the output threshold(s) applied in step 120 can have different values. In addition, in various other embodiments, different actuators can trigger step 120 over the course of operation of the surgical system.

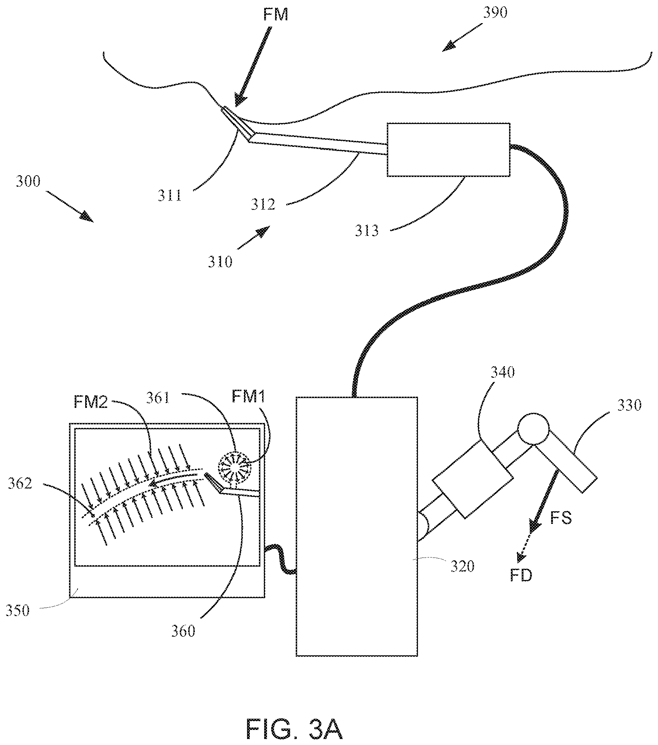

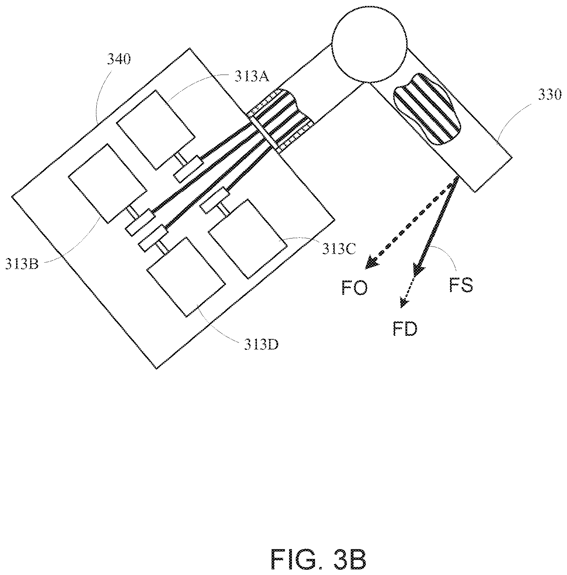

FIGS. 3A and 3B shows block diagram of a surgical system 300 incorporating haptic feedback at an input device 330 and means for providing a consistent haptic experience for the user as described above with respect to FIGS. 1, 2A, and 2B. Surgical system 300 includes an instrument 310 for performing a surgical task (e.g., forceps, cutter, retractor, vessel sealer, needle driver, catheter, etc.), an input device 330 (e.g., a lever(s), gripper(s), joystick(s), or any other structure capable of receiving user input) for receiving inputs from a user (e.g., surgeon), and a controller 320 for receiving input instructions from input device 330, controlling the actions of instrument 310 accordingly via a manipulation structure 313, and providing instructions to a haptic feedback actuation mechanism 340 to provide haptic feedback to input device 330 according to a desired haptic feedback profile. In various embodiments, manipulation structure 313 can include any number of systems and structures for maneuvering, positioning, actuating, or otherwise controlling the behavior of instrument 310, including a robotic arm(s)/manipulator(s), set up structure(s), and/or positioning element(s) such as a boom(s) or cart(s), among others. Controller 320 can include any combination of hardware, software, firmware, and other modalities for generating, managing, controlling, and effecting the actions described herein. In various embodiments, controller 320 can be integrated with instrument 310, input device 330, and/or discrete control hardware (e.g., a standalone processing unit or computing platform).

For exemplary purposes, FIG. 3A shows an end effector 311 at the end of a shaft 312 of instrument 310 grasping a portion of tissue 390 (e.g., retraction). This results in a force FM at end effector 311, which would ideally be delivered as a desired haptic feedback profile force FD at input device 330. The actual haptic feedback delivery is enabled by haptic feedback actuation mechanism 340, which includes multiple actuators that attempt to provide the haptic feedback profile force FD to present the surgeon with a "feel" of the resistance being provided by tissue 390 as it is being retracted.

Although the haptic feedback profile force FD is described as being derived from a force FM sensed at end effector 311 of instrument 310 for exemplary purposes, in various other embodiments, force FM could be sensed at any location for which corresponding haptic feedback at input device 330 would be beneficial, such as interactions at shaft 312 or any other element of manipulation structure 313 (e.g., arm collisions with structures or staff).

In various other embodiments, force FM can be defined according to non-physical parameters, such as guidance or user interface features. For example, in some embodiments, surgical system 300 can include a display 350 (e.g., a monitor(s), a head-in viewer(s), projections, video glasses/helmet(s), and/or any other graphical presentation element). In various embodiments, display 350 can present a virtual or synthetic element 361 that can be interacted with via input device 330. In some embodiments, synthetic element 361 can be used as a supplemental interface for interacting with a physical component of surgical system 300. For example, as shown in FIG. 3A, synthetic element 361 can be a virtual handle or knob that can be "grasped" and dragged around using input device 330 to reposition instrument 310 at the surgical site. In other embodiments, synthetic element 361 can provide a purely virtual interaction element, such as a dial, toggle, lever, or any other structure for controlling surgical system 300. In any case, by generating a haptic feedback profile based on model forces FM1 associated with interacting with synthetic element 361 (e.g., radially outward resistive force produced with grasping a round knob), controller 320 can then attempt to provide an appropriate haptic feedback profile force FD at input device 330.

In various other embodiments, surgical system 300 may provide guidance to the user with respect to movement of instrument 310 and/or input device 330. For example, a desired motion of instrument 310 (e.g., a targeted or safe dissection path, a desired retraction movement, or any other beneficial articulation) could optionally be defined as a trajectory 362. By generating a haptic feedback profile based on model forces FM2 associated with maintaining the position of instrument 310 along trajectory 362 (e.g., inwardly directed forces produced upon deviations from trajectory 362), controller 320 can then attempt to provide an appropriate haptic feedback profile force FD at input device 330.

FIG. 3B shows an exemplary block diagram of actuation mechanism 340 that includes multiple actuators 341 that apply components of the haptic feedback force to input device 330. Note that while four actuators that drive input device 330 via cables or tendons are depicted for descriptive purposes, in various other embodiments, actuation mechanism 340 can include any number and type of actuators (e.g., rotary actuators, linear actuators, hydraulics, and/or piezo-electric, vibrotactile, or fluidic actuators) and/or force transmission mechanisms (e.g., direct drive, linkages, gearing, etc.).

The various actuators 341 provide actuation outputs (e.g., torque or force) in combination with one another in an effort to produce the desired haptic feedback profile force FD. However, if desired haptic feedback profile force FD requires an output from one of actuators 341 that falls outside its working range, attempting to use the commanded outputs for actuators 341 without modification would result in an unmodified haptic feedback force FO having both a magnitude and direction different than desired force FD (as described above with respect to FIGS. 1 and 2A). As described above, the working range of an actuator can be defined by a maximum output threshold (e.g., at or below the output limit of the actuator) and/or a minimum output threshold (e.g., at or above a minimum reliable output level of the actuator).

Therefore, when controller 320 detects that a commanded output for any of actuators 341 would go beyond that actuator's defined output threshold (as described above with respect to step 120 in FIG. 1), it applies a common scaling factor to the commanded outputs supplied to each actuator 341 such that the commanded outputs for all actuators 341 remain within their predetermined working range (as described above with respect to step 130 in FIG. 1). This has the effect of changing the output of all actuators 341 proportionately, which in turn results in a scaled haptic feedback force FS that, while different in magnitude with respect to the desired feedback force FD, remains directionally aligned with desired feedback force FD (as described above with respect to FIG. 2B).

In various embodiments, when controller 320 detects that the unsealed commanded outputs would no longer go beyond the defined output thresholds for any of actuators 341, the scaling factor can be eliminated (or set to 1), thereby allowing restoration of unsealed haptic feedback at input device 330 (as described above with respect to steps 140 and 150 in FIG. 1).

## Claims

1. A surgical system (300) comprising:
a user input device (330);
a haptic feedback mechanism (340) including a first actuator coupled to the user input device (330) and a second actuator coupled to the user input device (330); and
a controller (320) coupled to receive input instructions from the user input device (330) and to provide instructions to the haptic feedback mechanism (340), the controller (320) being configured to determine a first commanded output haptic feedback force for the first actuator to provide to the user input device (330) and a second commanded output haptic feedback force for the second actuator to provide to the user input device (341), on a condition in which the first commanded output haptic feedback force would be greater than an upper output limit (OL1) of the first actuator, the controller (320) is configured to:
apply a common scaling factor to the first commanded output haptic feedback force to generate a scaled first commanded output feedback force for the first actuator to provide to the user input device (330), the common scaling factor adjusts the first commanded output haptic feedback force to be at or below the upper output limit (OL1) of the first actuator,
apply the common scaling factor to the second commanded output haptic feedback force to generate a scaled second commanded output feedback force for the second actuator to provide to the user input device (330), and
provide a user haptic feedback at the user input device (330) by concurrently providing the scaled first commanded output feedback force via the first actuator and the scaled second commanded output feedback force via the second actuator.

2. The surgical system (300) of claim 1, wherein:
a working range of the first actuator is defined at least in part by the upper output limit (OL1) of the first actuator and by a lower output limit of the first actuator; and
the lower output limit is at or above a minimum reliable output level force of the first actuator.

3. The surgical system (300) of claim 2, wherein:
the common scaling factor adjusts the first commanded output haptic feedback force to be within the working range.

4. The surgical system (300) of claim 2, wherein:
on a condition in which the first commanded output haptic feedback force would fall within the working range of the first actuator, the controller (320) removes the common scaling factor from the scaled first commanded output feedback force and the scaled second commanded output feedback force.

5. The surgical system (300) of claim 2, wherein:
the lower output limit is dynamic and based on a kinematic configuration of the user input device (330).

6. The surgical system (300) of claim 2, wherein:
a working range of the second actuator is defined at least in part by an upper output limit of the second actuator;
the common scaling factor adjusts the first commanded output haptic feedback force to be within the working range of the first actuator; and
the common scaling factor adjusts the second commanded output haptic feedback force to be within the working range of the second actuator.

7. The surgical system (300) of claim 6, wherein:
the working range of the second actuator is further defined by a lower output limit of the second actuator; and
the lower output limit of the second actuator is at or above a minimum reliable output level force of the second actuator.

8. The surgical system (300) of any of claims 6 or 7, wherein:
the condition in which the first commanded output haptic feedback force would be greater than the upper output limit (OL1) of the first actuator is a first condition;
on a second condition in which the first scaling factor applied to the first commanded output haptic feedback force would cause the second actuator to output a force outside the working range of the second actuator, the controller (320) applies a second scaling factor to the first commanded output haptic feedback force and to the second commanded output haptic feedback force; and
the second scaling factor adjusts the second commanded output haptic feedback force to be within the working range of the second actuator.

9. The surgical system (300) of any of claims 1-7, wherein:
the upper output limit (OL1) is dynamic and based on a kinematic configuration of the user input device (330).

10. The surgical system (300) of any of claims 1-7, wherein:
the scaled first commanded output feedback force and the scaled second commanded output feedback force are associated an instrument state

11. The surgical system (300) of any of claims 1-7, wherein:
the scaled first commanded output feedback force and the scaled second commanded output feedback force are associated an instrument speed.

12. The surgical system (300) of any of claims 1-7, wherein:
the scaled first commanded output feedback force and the scaled second commanded output feedback force are associated a viewing magnification.

13. The surgical system (300) of any of claims 1-7, wherein:
the common scaling factor is variable.

14. The surgical system (300) of any of claims 1-7, wherein:
the upper output limit (OL1) is associated with a maximum output threshold force of the first actuator.

## Patentansprüche

1. Chirurgisches System (300), das Folgendes umfasst:
ein Benutzereingabegerät (330);
einen haptischen Rückmeldungsmechanismus (340), einschließend einen ersten Aktuator, der mit dem Benutzereingabegerät (330) gekoppelt ist, und einen zweiten Aktuator, der mit dem Benutzereingabegerät (330) gekoppelt ist; und
eine Steuerung (320), die gekoppelt ist, um Eingabeanweisungen von dem Benutzereingabegerät (330) zu empfangen und Anweisungen an den haptischen Rückmeldungsmechanismus (340) bereitzustellen, wobei die Steuerung (320) zum Bestimmen einer ersten befohlenen haptischen Ausgangsrückmeldungskraft für den ersten Aktuator zum Geben an das Benutzereingabegerät (330) und einer zweiten befohlenen haptischen Ausgangsrückmeldungskraft für den zweiten Aktuator zum Geben an das Benutzereingabegerät (341) unter einer Bedingung konfiguriert ist, in der die erste befohlene ausgegebene haptische Rückmeldungskraft größer als eine obere Ausgangsgrenze (OL1) des ersten Aktuators wäre, wobei die Steuerung (320) konfiguriert ist zum:
Anwenden eines gemeinsamen Skalierfaktors auf die erste befohlene haptische Ausgangsrückmeldungskraft, um eine skalierte erste befohlene Ausgangsrückmeldungskraft für den ersten Aktuator zum Geben an das Benutzereingabegerät (330) zu erzeugen, wobei der gemeinsame Skalierfaktor die erste befohlene haptische Ausgangsrückmeldungskraft so einstellt, dass sie bei oder unter der oberen Ausgangsgrenze (OL1) des ersten Aktuators liegt,
Anwenden des gemeinsamen Skalierfaktors auf die zweite befohlene haptische Ausgangsrückmeldungskraft, um eine skalierte zweite befohlene Ausgangsrückmeldungskraft für den zweiten Aktuator zum Geben an das Benutzereingabegerät (330) zu erzeugen, und
Geben einer haptischen Benutzerrückmeldung an dem Benutzereingabegerät (330) durch gleichzeitiges Geben der skalierten ersten befohlenen Ausgangsrückmeldungskraft mittels des ersten Aktuators und der skalierten zweiten befohlenen Ausgangsrückmeldungskraft mittels des zweiten Aktuators.

2. Chirurgisches System (300) nach Anspruch 1, wobei:
ein Arbeitsbereich des ersten Aktuators zumindest teilweise durch die obere Ausgangsgrenze (OL1) des ersten Aktuators und durch eine untere Ausgangsgrenze des ersten Aktuators definiert ist; und
die untere Ausgangsgrenze bei oder über einer minimal zuverlässigen Ausgangsniveaukraft des ersten Aktuators liegt.

3. Chirurgisches System (300) nach Anspruch 2, wobei:
wobei der gemeinsame Skalierfaktor die erste befohlene haptische Ausgangsrückmeldungskraft so einstellt, dass sie innerhalb des Arbeitsbereichs liegt.

4. Chirurgisches System (300) nach Anspruch 2, wobei:
unter einer Bedingung, in der die erste befohlene haptische Ausgangsrückmeldungskraft innerhalb des Arbeitsbereichs des ersten Aktuators fallen würde, die Steuerung (320) den gemeinsamen Skalierfaktor von der skalierten ersten befohlenen Ausgangsrückmeldungskraft und der skalierten zweiten befohlenen Ausgangsrückmeldungskraft entfernt.

5. Chirurgisches System (300) nach Anspruch 2, wobei:
die untere Ausgangsgrenze dynamisch ist und auf einer kinematischen Konfiguration des Benutzereingabegeräts (330) basiert.

6. Chirurgisches System (300) nach Anspruch 2, wobei:
ein Arbeitsbereich des zweiten Aktuators zumindest teilweise durch eine obere Ausgangsgrenze des zweiten Aktuators definiert ist;
der gemeinsame Skalierfaktor die erste befohlene haptische Ausgangsrückmeldungskraft so einstellt, dass sie innerhalb des Arbeitsbereichs des ersten Aktuators liegt; und
der gemeinsame Skalierfaktor die zweite befohlene haptische Ausgangsrückmeldungskraft so einstellt, dass sie innerhalb des Arbeitsbereichs des zweiten Aktuators liegt.

7. Chirurgisches System (300) nach Anspruch 6, wobei:
der Arbeitsbereich des zweiten Aktuators ferner durch eine untere Ausgangsgrenze des zweiten Aktuators definiert ist; und
die untere Ausgangsgrenze des zweiten Aktuators bei oder über einer minimal zuverlässigen Ausgangsniveaukraft des zweiten Aktuators liegt.

8. Chirurgisches System (300) nach einem der Ansprüche 6 oder 7, wobei:
die Bedingung, in der die erste befohlene haptische Ausgangsrückmeldungskraft größer als die obere Ausgangsgrenze (OL1) des ersten Aktuators wäre, eine erste Bedingung ist;
unter einer zweiten Bedingung, in der der erste Skalierfaktor, der auf die erste befohlene haptische Ausgangsrückmeldungskraft angewendet wird, bewirken würde, dass der zweite Aktuator eine Kraft außerhalb des Arbeitsbereichs des zweiten Aktuators ausgibt, die Steuerung (320) einen zweiten Skalierfaktor auf die erste befohlene haptische Ausgangsrückmeldungskraft und auf die zweite befohlene haptische Ausgangsrückmeldungskraft anwendet; und
der zweite Skalierfaktor die zweite befohlene haptische Ausgangsrückmeldungskraft so einstellt, dass sie innerhalb des Arbeitsbereichs des zweiten Aktuators liegt.

9. Chirurgisches System (300) nach einem der Ansprüche 1-7, wobei:
die obere Ausgangsgrenze (OL1) dynamisch ist und auf einer kinematischen Konfiguration des Benutzereingabegeräts (330) basiert.

10. Chirurgisches System (300) nach einem der Ansprüche 1-7, wobei:
die skalierte erste befohlene Ausgangsrückmeldungskraft und die skalierte zweite befohlene Ausgangsrückmeldungskraft mit einem Instrumentenzustand assoziiert sind.

11. Chirurgisches System (300) nach einem der Ansprüche 1-7, wobei:
die skalierte erste befohlene Ausgangsrückmeldungskraft und die skalierte zweite befohlene Ausgangsrückmeldungskraft mit einer Instrumentengeschwindigkeit assoziiert sind.

12. Chirurgisches System (300) nach einem der Ansprüche 1-7, wobei:
die skalierte erste befohlene Ausgangsrückmeldungskraft und die skalierte zweite befohlene Ausgangsrückmeldungskraft mit einer Betrachtungsvergrößerung assoziiert sind.

13. Chirurgisches System (300) nach einem der Ansprüche 1-7, wobei:
der gemeinsame Skalierfaktor variabel ist.

14. Chirurgisches System (300) nach einem der Ansprüche 1-7, wobei:
die obere Ausgangsgrenze (OL1) mit einer maximalen Ausgangsschwellenwertkraft des ersten Aktuators assoziiert ist.

## Revendications

1. Système chirurgical (300) comprenant :
un dispositif d'entrée d'utilisateur (330) ;
un mécanisme de rétroaction haptique (340) incluant un premier actionneur couplé au dispositif d'entrée d'utilisateur (330) et un deuxième actionneur couplé au dispositif d'entrée d'utilisateur (330) ; et
un dispositif de commande (320) couplé pour recevoir des instructions d'entrée du dispositif d'entrée d'utilisateur (330) et pour fournir des instructions au mécanisme de rétroaction haptique (340), le dispositif de commande (320) étant configuré pour déterminer une première force de rétroaction haptique de sortie commandée pour que le premier actionneur la fournisse au dispositif d'entrée d'utilisateur (330) et une deuxième force de rétroaction haptique de sortie commandée pour que le deuxième actionneur la fournisse au dispositif d'entrée d'utilisateur (341), dans une condition dans laquelle la première force de rétroaction haptique de sortie commandée serait supérieure à une limite de sortie supérieure (LS1) du premier actionneur, le dispositif de commande (320) est configuré pour :
appliquer un facteur d'échelle commun à la première force de rétroaction haptique de sortie commandée pour générer une première force de rétroaction de sortie commandée mise à l'échelle pour que le premier actionneur la fournisse au dispositif d'entrée d'utilisateur (330), le facteur d'échelle commun ajuste la première force de rétroaction haptique de sortie commandée devant être au niveau ou au-dessous de la limite de sortie supérieure (LS1) du premier actionneur,
appliquer le facteur d'échelle commun à la deuxième force de rétroaction haptique de sortie commandée pour générer une deuxième force de rétroaction de sortie commandée mise à l'échelle pour que le deuxième actionneur la fournisse au dispositif d'entrée d'utilisateur (330), et
fournir une rétroaction haptique d'utilisateur au niveau du dispositif d'entrée d'utilisateur (330) en fournissant de manière simultanée la première force de rétroaction de sortie commandée mise à l'échelle via le premier actionneur et la deuxième force de rétroaction de sortie commandée mise à l'échelle via le deuxième actionneur.

2. Système chirurgical (300) selon la revendication 1, dans lequel :
une plage de travail du premier actionneur est définie au moins en partie par la limite de sortie supérieure (LS1) du premier actionneur et par une limite de sortie inférieure du premier actionneur ; et
la limite de sortie inférieure est au niveau ou au-dessus d'une force de niveau de sortie fiable minimum du premier actionneur.

3. Système chirurgical (300) selon la revendication 2, dans lequel :
le facteur d'échelle commun ajuste la première force de rétroaction haptique de sortie commandée pour qu'elle soit dans les limites de la plage de travail.

4. Système chirurgical (300) selon la revendication 2, dans lequel :
dans une condition dans laquelle la première force de rétroaction haptique de sortie commandée tomberait dans les limites de la plage de travail du premier actionneur, le dispositif de commande (320) retire le facteur d'échelle commun de la première force de rétroaction de sortie commandée mise à l'échelle et de la deuxième force de rétroaction de sortie commandée mise à l'échelle.

5. Système chirurgical (300) selon la revendication 2, dans lequel :
la limite de sortie inférieure est dynamique et basée sur une configuration cinématique du dispositif d'entrée d'utilisateur (330).

6. Système chirurgical (300) selon la revendication 2, dans lequel :
une plage de travail du deuxième actionneur est définie au moins en partie par une limite de sortie supérieure du deuxième actionneur ;
le facteur d'échelle commun ajuste la première force de rétroaction haptique de sortie commandée pour qu'elle soit dans les limites de la plage de travail du premier actionneur ; et
le premier facteur d'échelle commun ajuste la deuxième force de rétroaction haptique de sortie commandée pour qu'elle soit dans les limites de la plage de travail du deuxième actionneur.

7. Système chirurgical (300) selon la revendication 6, dans lequel :
la plage de travail du deuxième actionneur est définie en outre par une limite de sortie inférieure du deuxième actionneur ; et
la limite de sortie inférieure du deuxième actionneur est au niveau ou au-dessus d'une force de niveau de sortie fiable minimum du deuxième actionneur.

8. Système chirurgical selon (300) l'une quelconque des revendications 6 ou 7, dans lequel :
la condition dans laquelle la première force de rétroaction haptique de sortie commandée serait supérieure à la limite de sortie supérieure (LS1) du premier actionneur est une première condition ;
dans une deuxième condition dans laquelle le premier facteur d'échelle appliqué à la première force de rétroaction haptique de sortie commandée amènerait le deuxième actionneur à sortir une force en dehors de la plage de travail du deuxième actionneur, le dispositif de commande (320) applique un deuxième facteur d'échelle à la première force de rétroaction haptique de sortie commandée et à la deuxième force de rétroaction haptique de sortie commandée ; et
le deuxième facteur d'échelle ajuste la deuxième force de rétroaction haptique de sortie commandée pour qu'elle soit dans les limites de la plage de travail du deuxième actionneur.

9. Système chirurgical (300) selon l'une quelconque des revendications 1 à 7, dans lequel :
la limite de sortie inférieure (LS1) est dynamique et basée sur une configuration cinématique du dispositif d'entrée d'utilisateur (330).

10. Système chirurgical (300) selon l'une quelconque des revendications 1 à 7, dans lequel :
un état d'instrument est associé à la première force de rétroaction de sortie commandée mise à l'échelle et à la deuxième force de rétroaction de sortie commandée mise à l'échelle

11. Système chirurgical (300) selon l'une quelconque des revendications 1 à 7, dans lequel :
une vitesse d'instrument est associée à la première force de rétroaction de sortie commandée mise à l'échelle et à la deuxième force de rétroaction haptique de sortie commandée mise à l'échelle.

12. Système chirurgical (300) selon l'une quelconque des revendications 1 à 7, dans lequel :
un grossissement de visualisation est associé à la première force de rétroaction de sortie commandée mise à l'échelle et à la deuxième force de rétroaction de sortie commandée mise à l'échelle.

13. Système chirurgical (300) selon l'une quelconque des revendications 1 à 7, dans lequel :
le facteur de mise à l'échelle commun est variable.

14. Système chirurgical (300) selon l'une quelconque des revendications 1 à 7, dans lequel :
la limite de sortie supérieure (LS1) est associée à une force de seuil de sortie maximum du premier actionneur.
